# EUROPEAN PATENT SPECIFICATION

(11) **EP 0 458 849 B1**
(45) Date of publication and mention of the grant of the patent: **03.05.1995**
(21) Application number: 90903330.0
(22) Date of filing: 16.02.1990
(51) Int. Cl.: C12N 9/98, C11D 3/386

(54) **ENZYME CONTAINING GRANULATE AND METHOD FOR PRODUCTION THEREOF**
ENZYM ENTHALTENDES GRANULAT UND VERFAHREN ZUR HERSTELLUNG
GRANULE CONTENANT DES ENZYMES ET PROCEDE DE PRODUCTION D'UN TEL GRANULE

(30) Priority: 20.02.1989 DK 781/89
(43) Date of publication of application: 04.12.1991
(73) Proprietor: NOVO NORDISK A/S, 2880 Bagsvaerd (DK)
(72) Inventor: MARKUSSEN, Erik, Kjaer, DK-3500 Vaerloese (DK); FALHOLT, Per, DK-2900 Hellerup (DK)
(74) Representative: Bach, Niels
(86) International application number: DK9000042
(87) International publication number: WO9009440

(56) References cited:
- EP-A- 0 271 153
- EP-A- 0 286 773
- EP-A- 0 304 332
- WO-A-89/08694
- US-A- 4 740 469

## Description

This invention relates to an enzyme containing granulate and a method for production thereof.

The granulate according to the invention and a method for production thereof is a further development of the granulate and the method for production thereof described in EP-A-0 304 332, which is carried out in a drum granulator. It is to be understood that the term drum granulator represents a broad category of granulator devices, comprising for instance pan granulators and granulation mixers. The granulate according to the invention, however, can also be produced by means of other granulating devices than drum granulators, as will appear later in this specification. Also, the "double enzyme" granulate is advantageous for the soaper, as it reduces the number of additive outlets in the soaper's dosing system.

The enzyme containing granulate described in EP-A-0 304 332 comprises a core surrounded by a coating comprising cellulose fibres or artificial fibres in an amount of 1.5 to 40% by weight, based on the dry weight of the granulate except for the core, a binder in an amount of 0 to 15% by weight, based on the dry weight of the granulate, except for the core, a filler, and one or more granulating agents, whereby the core and/or the coating contains an enzyme.

The invention is a further development of the special embodiment of the invention described in EP-A-0 304 332, which comprises a granulate with an enzyme in the core (here identified as the first enzyme) and another enzyme in the shell (here identified as the second enzyme). In those cases where the second enzyme is sensitive to the first enzyme (the second enzyme could for instance be a lipase, and the first enzyme could be a protease, whereby the lipase could have a tendency to be broken down by the protease),there is a need for a fast release of the second enzyme in the washing solution and a slow release of the first enzyme in the washing solution.

Thus, the purpose of the invention is the provision of an enzyme containing granulate which contains a first enzyme and a second enzyme sensitive to the first enzyme, and whereby the second enzyme is released fast in the washing solution, whereas the first enzyme is released slowly in the washing solution. Also, the "double enzyme" granulate is advantageous for the soaper, as it reduces the number of additive outlets in the soaper's dosing system.

The enzyme containing granulate according to the invention comprises a core with a first enzyme and cellulose fibres or artificial fibres in an amount of between 1.5 and 40% by weight, based on the weight of the core, surrounded by a shell comprising a binder, a filler, granulating agents, and a second enzyme, and a sustained release coating between the core and the shell, the second enzyme being sensitive to the first enzyme. The cellulose fibres or artificial fibres are preferably present in an amount of between 5 and 20% by weight, based on the weight of the core.

Co-pending European application No. 90903319.3 (EP-A-0 458 845, with the same filing date as this application) describes a related, but different invention, as arbitrary detergent additives (i.e. not only enzymes) can be present in the core and the shell of the described detergent additive granulate.

US patent no. 4740469 describes an enzyme granulate which contains 0.5-30 weight-% of synthetic fibres, and which is coated with a waxy material and a colorant. However, in contradistinction thereto, in the enzyme containing granulate according to the invention the coated core is surrounded by a shell comprising a binder, a filler, granulating agents and a second enzyme.

Due to the sustained release coating between the core and the shell the second enzyme is released fast in the washing solution and the first enzyme is released slowly.

The core material, the cellulose fibres or the artificial fibres, the binder, the filler, the granulating agent, and the facultative additives can be of the same kind and used in the same way as described in EP-A-0 304 332. However, whereas the cellulose fibres or the artificial fibres are a part of the shell in the granulate of EP-A-0 304 332, they are a part of the core in this invention.

The sustained release coating can be any sustained release coating used in the art, which do not hurt the first or second enzymes or in other manner disturb the manufacturing process or the application of the granulate. Examples of usable sustained release coating agents are those described in European patent No. 0270608, International patent publication No. WO 89/08694, and in C.A. Finch: Polymers for microcapsule walls, Chemistry and Industri, November 18, 1985 (p. 752).

Examples of enzyme pairs (whereby enzymes are indicated in the sequence (first enzyme) - (second enzyme)) are the following: protease-lipase, protease-cellulase, protease-amylase, protease-peroxidase, protease-amidase, protease-oxidase, and protease-protease. The second enzyme in the enzyme pair protease-protease could be a protease, which is sensitive to ALCALASE® or SAVINASE® or ESPERASE®, whereby a synergistic effect could be obtained by means of the combination of first and second protease. Generally the first enzyme is usually a protease and the second enzyme is generally a protease labile enzyme.

In a preferred embodiment of the granulate according to the invention the core is of a shape corresponding to a maximum ratio between the largest and the smallest dimension of around 3. This maximum ratio is preferably around 2, more preferably around 1.5. In this embodiment a satisfactory coating around the core can always be obtained.

In a preferred embodiment of the granulate according to the invention the amount of the core is between 5 and 75% by weight. The amount of the core is preferably between 10 and 50% by weight of the granulate, most preferably between 15 and 40% by weight of the granulate. If the amount of the core is above 75%, the loading of a sufficient amount of second enzyme will be difficult, and if the amount of the core is less than 5%, loading of a sufficient amount of first enzyme will be difficult.

In a preferred embodiment of the granulate according to the invention the core has a mean particle size between 100 and 1000 »m. This mean particle size is preferably 200 - 700 »m. These are the particle sizes most often required by the users.

In a preferred embodiment of the granulate according to the invention the cellulose fibres or the artificial fibres have an average fibre length of 50-2000 »m and an average fibre width of 5 - 50 »m. The average fibre length is preferably 100-1000 »m and the average fibre width is preferably 10 - 40 »m. Hereby a satisfactory mechanical strength is obtained.

In a preferred embodiment of the granulate according to the invention the shell contains between 1.5 and 40% by weight of cellulose fibres or artificial fibres. Hereby the physical strength of the granulate is improved.

In a preferred embodiment of the granulate according to the invention the first enzyme is ALCALASE®, SAVINASE®, and/or ESPERASE® and the second enzyme is a lipase. A satisfactory sustained release effect during washing will be obtained.

In a preferred embodiment of the granulate according to the invention the first enzyme is ALCALASE®, SAVINASE®, and/or ESPERASE® and the second enzyme is LIPOLASE®. A most satisfactory sustained release effect during washing has been observed.

In a preferred embodiment of the granulate according to the invention the filler consists of or comprises inorganic salts. Hereby a cheap granulate is obtained.

In a preferred embodiment of the granulate according to the invention the shell is surrounded by a final dust suppressing coating. This is advantageous in those cases where the shell tends to produce an unacceptable amount of dust.

In a preferred embodiment of the granulate according to the invention the sustained release coating is a material comprising a mono- and/or di- and/or triglyceride of a fatty acid or fatty acids. It has been found that in this embodiment a most satisfactory sustained release action during the washing process can be obtained.

Also, the invention comprises a method for production of the granulate according to the invention.

The method for the production of the enzyme containing granulate according to the invention comprises the introduction of the core into a granulating device, whereafter sequentially the sustained release coating agent, the shell material and optionally a final dust suppressing coating agent are introduced into the granulating device.

It is to be understood that any granulating device can be used in relation to the method according to the invention, e.g. a granulating drum, a fluid bed coater or the granulating device described in WO 85/05288. The entire process can be carried out in one of these granulating devices. Also, it is to be understood that the core can be coated with the sustained release coating agent in one granulating device, whereafter the coated core can be isolated an transferred to another granulating device for application of the shell, whereafter if wanted application of a dust suppression coating can be performed on the same granulating device or another granulating device.

The method according to the invention can be performed both batchwise and continuously.

The invention will be illustrated by the following examples.

The activity units used in the examples are defined as follows.

| enzyme | activity unit | definition indicated in |
|---|---|---|
| proteolytic | Anson | AF 4.3/5-GB |
| | KNPU | AF 220/1-GB |
| amylolytic | KNU | AF 215/1-GB |
| lipolytic | LU | AF 95/4-GB |
| cellulolytic | CSU | AF 253/2-GB |

On request these publicly available AF publications can be obtained from NOVO NORDISK A/S, Novo Allé, 2880 Bagsvaerd, Denmark.

The Examples I, II, and III illustrate the preparation of three granulates according to the invention, and the three corresponding products are identified as granulate I, II, and III. These three products are used in the application Examples 1 and 2.

### Preparation examples

### EXAMPLE I

- Step 1.: A granulate with proteolytic activity and with the composition
- Core: 25% ALCALASE® concentrate (15,1 AU/g)
15% fibrous cellulose, ARBOCEL BC200
4% kaolin, type ECC Speswhite
10% carbohydrate binder
46% finely ground sodium sulphate
is produced and dried as described in US patent No. 4,106,991, Example 1. The dried granulate with an activity of 3.6 AU/g is sifted and the fraction between 300 »m and 710 »m is further treated as described below in step 2.
- Step 2.: Coating with a sustained release layer of glyceryl stearate/palmitate Grindtek MSP90 (melting point 68°C) and kaolin (Speswhite, EEC).
- Sustained release layer: 7 kg of sifted granulate according to step 1 is heated in a 20 l Lödige mixer to 75-80°C which temperature is kept during the coating process. The coating is applied with continuous mixing and alternately applying melted MSP90 and kaolin in such balanced way that the charge is neither too sticky neither contains a substantial amount of free kaolin powder. In the example the actual sequences and amounts of materials are:
1. 500 g MSP90
2. 1000 g kaolin
3. 300 g MSP90
4. 1000 g kaolin
5. 200 g MSP90
6. 1000 g kaolin
7. 200 g MSP90
8. 1000 g kaolin
9. 200 g MSP90
10. 1000 g kaolin
Total: 1.4 kg MSP90 and 5.0 kg kaolin.
Step 2 is completed by cooling the coated granulate on a fluid bed to room temperature and a minor amount (<1%) of non product material is sifted away on a 1.2 mm screen.
The coated granulate has a proteolytic activity of 1.85 AU/g.
- Step 3.: 10 kg of the granulate with a sustained release coating from step 2 is coated with a shell containing the lipolytic enzyme SP 341, which is the
- Shell: *Pseudomonas cepacia* lipase described in European patent application with publication No. 0214761:
20 kg of shell material with the composition:
30% SP 341 concentrate (lipolytic activity 195,000 LU/g)
15% fibrous cellulose ARBOCEL BC200
4% kaolin, ECC Speswhite
51% finely ground sodium sulphate
is mixed with and layered on the surface of the 10 kg of granulate from step 2 as described in Danish patent application No. 4356/87 (equivalent to Danish patent No. 157937), Example 1 except that in the present example the core has a proteolytic activity and the shell has a lipolytic activity.
Step 3 is concluded with a fluid bed drying of the granulate to a water content below 1% and sifting between 300 »m and 1200 »m.
- Step 4.: Coating with 4% PEG 4000 and 10% of a mixture of titanium dioxyde:kaolin 1:1 as described in US patent No. 4,106,991, Example 22.
The product after step 4 has a proteolytic activity of 0.53 AU/g and a lipolytic activity of 31,000 LU/g.

### EXAMPLE II

- Step 1.: A granulate with proteolytic activity and with the composition:
22% SAVINASE® concentrate (51 KNUP/g)
15% fibrous cellulose, ARBOCEL BC200
4% kaolin, type ECC Speswhite
10% carbohydrate binder
49% finely ground sodium sulphate
is produced as described in Example I, step 1.
The proteolytic activity of the dried granulate is 10.8 KNPU/g.
- Step 2.: A sustained release coating is applied as described in Example I, step 2.
The coated granulate has a proteolytic activity of 5.4 KNPU/g.
- Step 3.: A lipolytic shell with enzyme SP 341 is applied as described in Example I, step 3.
- Step 4.: A coating is applied as described in Example I, step 4.
The product after step 4 has a proteolytic activity of 1.54 KNPU/g and a lipolytic activity of 31,300 LU/g.

### EXAMPLE III

- Step 1.: A granulate with proteolytic activity and with the composition:
34% ALCALASE® concentrate (15.1 AU/g)
15% fibrous cellulose, ARBOCEL BC200
4% kaolin, type ECC Speswhite
8% carbohydrate binder
39% finely ground sodium sulphate
is produced as described in Example I, step 1.
The proteolytic activity of the dried granulate is 4.9 AU/g.
- Step 2.: A sustained release coating is applied as described in Example I, step 2.
The coated granulate has a proteolytic activity of 2.5 AU/g.
- Step 3.: 10 kg of granulate with sustained release coating from step 2 is coated with a shell containing the lipolytic enzyme LIPOLASE®, which is the recombinant *Humicola* lipase described in European patent application with publication No. 0305216.
10 kg of shell material with the composition :
23% LIPOLASE® concentrate (lipolytic activity 680,000 LU/g)
15% fibrous cellulose ARBOCEL BC200
4% kaolin, ECC Speswhite
10% carbohydrate binder
48% finely ground sodium sulphate
is mixed with and layered on the surface of the 10 kg of granulate from step 2 as described in Danish patent application No. 4356/87 (equivalent to Danish patent No. 157937), except that in the present example the core has a proteolytic activity and the shell has a lipolytic activity, and that a 50 l Lödige mixer FM 50 was used.
Step 3 is concluded with a fluid bed drying of the granulate to a water content below 1% and sifting between 300 »m and 1200 »m.
- Step 4.: A coating is applied as described in Example I, step 1. The product after step 4 has a proteolytic activity of 1.08 AU/g and a lipolytic activity of 63,000 LU/g.

### EXAMPLE IV

- Step 1.: A Savinase® granulate is produced as described in Example II, step 1, except that the amount of fibrous cellulose in the core is 10% and the amount of carbohydrate binder 8%.
The proteolytic activity of the dried granulate is 7.8 KNPU/g.
- Step 2.: 10 kg of core material from step 1, sifted to obtain particle sizes between 400 and 700 »m, is transferred to a 20 liter Lödge mixer and provided with a sustained release coating of MSP 90 and kaolin as described in Example I, step 2.
Total: 1.0 kg MSP90 and 3.2 kg kaolin.
- Step 3.: 10 kg of the granulate with the sustained release coating from step 2 is coated with a shell containing the cellulolytic enzyme Celluzyme®, which is a commercial cellulase preparation from Novo Nordisk A/S.
10 kg of shell material with the composition:
50% Celluzyme® concentrate
10% fibrous cellulose, ARBOCEL BC200
4% kaolin, type ECC Speswhite
8% carbohydrate binder
28% finely ground sodium sulphate
is mixed with and layered on the surface of the 10 kg of granulate from step 2 in the manner described in Example I, step 3, except that the core has a proteolytic activity and the shell a cellulolytic activity.
The product after step 3 has a proteolytic activity of 3.5 KNPU/g and a cellulolytic activity of 750 CSU/g.

A washing experiment with this granulate was carried out in comparison to a control comprising a mixture of a Savinase® granulate and a Celluzyme® granulate, with comparable activities. It turned out that the cellulase activity/performance in relation to the washing experiment with the granulate according to the invention was higher/better than the cellulase activity/performance in relation to the control.

### EXAMPLE V

- Step 1.: A Savinase® granulate is produced as described in Example IV, step 1.
The proteolytic activity of the dried granulate is 7.8 KNPU/g.
- Step 2.: 10 kg of core material from step 1, sifted to obtain particle sizes between 400 and 700 »m, is transferred to a 20 liter Lödige mixer and provided with a sustained release coating of hydrogenated tallow with melting point 59°C and kaolin as described in Example I, step 2.
Total: 0.3 kg of hydrogenated tallow and 1.0 kg of kaolin.
A further top coating of a hydrophilic nature consisting of 0.5 kg PEG 4000 and 1.7 kg kaolin is applied in the manner described in Example I, step 2.
- Step 3.: A cellulolytic shell with the enzyme Celluzyme® is applied as described in Example IV, step 3.
The product after step 3 has a proteolytic activity of 3.8 KNPU/g and a cellulolytic activity of 530 CSU/g.

### EXAMPLE VI

- Step 1.: A Savinase® granulate is produced as described in Example IV, step 1.
The proteolytic activity of the dried granulate is 7.8 KNPU/g.
- Step 2.: 10 kg of core material from step 1, sifted to obtain particle sizes between 300 and 1200 »m, is transferred to a 20 liter Lödige mixer and provided with a sustained release coating of hydrogenated tallow and kaolin as described in Example I, step 2.
Total: 1.0 kg of hydrogenated tallow and 3.7 kg kaolin.
- Step 3.: 10 kg of the granulate with the sustained release coating from step 2 is coated with a shell containing the lipolytic enzyme Lipolase®, which is the recombinant *Humicola* lipase described in European patent application with publication No. 0 305 216.
10 kg of shell material with the composition:
1.5 kg fibrous cellulose, ARBOCEL BC200
1.0 kg bentonite, Type ASB350, ECC
1.0 kg carbohydrate binder
6.0 kg finely ground sodium sulphate
is mixed with and layered on the surface of granulate from step 2 by spraying with 0.9 kg of a liquid Lipolase® concentrate, produced by ultrafiltration and evaporation, in the manner described in Danish patent application No. 4356/87 (equivalent to Danish patent No. 157937), Example 1, except that in the present example the core has a proteolytic activity and the shell a lipolytic activity.
The product after step 3 has a proteolytic activity of 2.7 KNPU/g and a lipolytic activity of 7100 LU/g.

### EXAMPLE VII

- Step 1.: A granulate with proteolytic activity is produced as described in Example I, step 1, except that the enzyme is ESPERASE® and except that the amount of fibrous cellulose in the core is 10% and the amount of carbohydrate binder is 8%.
The proteolytic activity of the dried granulate is 8.5 KNPU/g.
- Step 2.: 10 kg of core material from step 1, sifted to obtain particle sizes between 300 and 1200 »m, is transferred to a 20 liter Lödige mixer and provided with a sustained release coating of hydrogenated fatty acid (C16/C18) with melting point 58°C and kaolin as described in Example I, step 2.
Total: 0.8 kg of hydrogenated fatty acid and 2.4 kg kaolin.
- Step 3.: 10 kg of the granulate with the sustained release coating from step 2 is coated with a shell containing an amylolytic enzyme BAN, which is a commercial bacterial amylase preparation from Novo Nordisk A/S.
10 kg of shell material with the composition:
0.2 kg BAN concentrate
1.0 kg fibrous cellulose, ARBOCEL BC200
0.4 kg kaolin, type ECC Speswhite
6.4 kg sodium sulphate
is mixed with and layered on the surface of the 10 kg of granulate from step 2 by spraying with 1.4 kg of a carbohydrate binder solution (40% in water). Otherwise the layering is performed as described in Example I, step 3.
The product after step 3 has a proteolytic activity of 4.3 KNPU/g and an amylolytic activity of 37 KNU/g.

### EXAMPLE VIII

- Step 1.: An Alcalase® granulate is produced as described in Example I, step 1, except that the amount of fibrous cellulose in the core is 10% and the amount of carbohydrate binder is 8%. The proteolytic activity of the dried granulate is 2.6 AU/g.
- Step 2.: 10 kg of core material from step 1, sifted to obtain particle sizes between 300 and 1200 »m, is transferred to a 20 liter Lödige mixer and provided with a sustained release coating of hydrogenated tallow with melting point 59°C and kaolin in the manner described in Example I, step 2.
Total: 0.8 kg of hydrogenated tallow and 2.2 kg kaolin.
- Step 3.: A cellulolytic shell with the enzyme Celluzyme® is applied as described in Example IV, step 3.
The product after step 3 has a proteolytic activity of 1.0 AU/g and a cellulolytic activity of 760 CSU/g.

A washing experiment with this granulate was carried out in comparison to a control comprising a mixture of an Alcalase® granulate and a Celluzyme® granulate, with comparable activities. It turned out that the cellulase activity/performance in relation to the invention was higher/better than the cellulase activity/performance in relation to the control.

### Application examples

The washing performance examples are made with a detergent of a composition as listed below:

| | |
|---|---|
| LAS (Nansa S80/S) | 6.5% |
| Soap (Vitra C.) | 2.0% |
| AE (Berol 160) | 3.5% |
| STPP (Janssen) | 26.0% |
| Sodium carbonate | 5.0% |
| Sodium sulphate | 21.0% |
| Sodium metasilicate (BHD 26186) | 8.0% |
| Sodium perborate (Fluka 71480) | 14.0% |
| TAED | 1.5% |
| EDTA | 0.2% |
| Water | to 100% |

The lipase efficacy is checked in a 4-cycle-soil-wash procedure. After the wash the swatches are dried at room temperature for 16 hours and then resoiled and stored 1-3 days before the next wash. The detergent dosage is 5 g/l and the following conditions are used:

| | | |
|---|---|---|
| Machine: | TergoTometer | Temperature: 30°C |
| Water: | 18° dH | Swatch/wash liquid: 1 swatch/l |
| pH: | 10.0 | Textile: cotton |
| Time: | 30 minutes | Swatch size: 7x50 cm |

Soiling: The swatches are soiled with a combination of a lipid/protein/clay emulsion of following composition:

| | |
|---|---|
| Olive oil (Sigma 01500) | 14.4% |
| Stearic acid (Sigman S 4876) | 1.8% |
| Monoglyceride (Grindtek MSP 90) | 1.8% |
| Gelatine (Oxoid) | 0.9% |
| Kaolin (Sigma K7375) | 1.4% |
| Carbon black (Degussa, Spezial schwarz 4) | 0.2% |
| Indian ink (Rotring) | 0.2% |
| Water | 79.4% |

### Evaluation

Remission of the swatches are measured at 460 nm after each wash. The residual fatty matter content is determined gravimetrically after a Soxhlet extraction (5 hours, hexane).

### Dosage

The enzymatic activities are dosed at a level equivalent to the reference examples.

### EXAMPLE 1

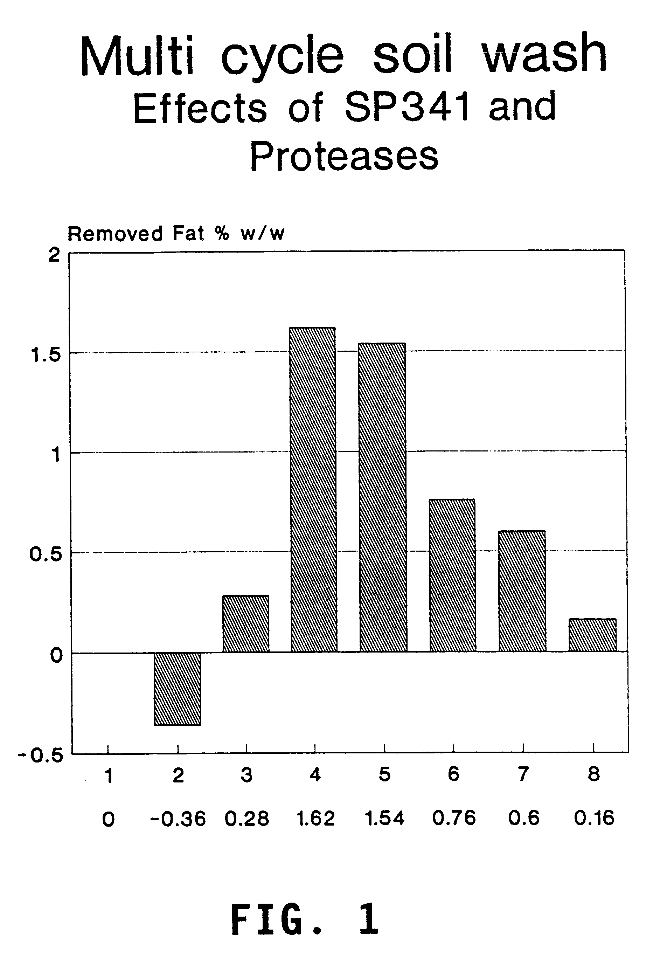

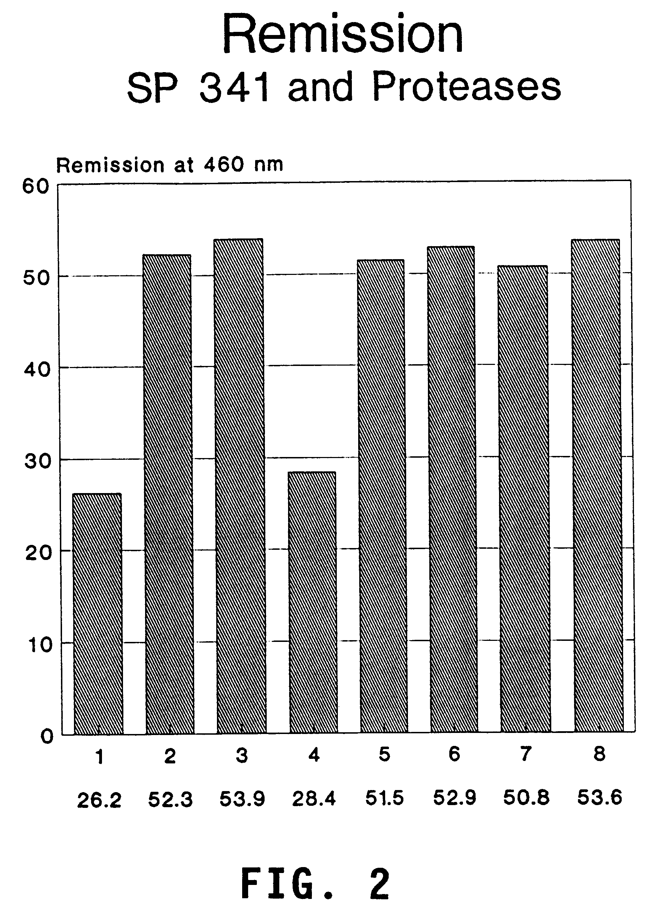

The results from the performance test are listed in Figs. 1 and 2. Fig. 1 shows the lipase efficacy whereas Fig. 2 shows the overall performance measured as remission of the swatches.

The significance of the upper numerals along the abscissa on Fig. 1 is as follows:
1. Detergent
2. Detergent + Alcalase® 2.0 T (0.05 AU/l)
3. Detergent + Savinase® 4.0 T (0.15 KNPU/l)
4. Detergent + SP 341 (3000 LU/l)
5. Detergent + Granulate I (Alcalase®/SP 341)
6. Detergent + SP 341 + Alcalase® 2.0 T
7. Detergent + Granulate II (Savinase®/SP 341)
8. Detergent + SP 341 + Savinase® 4.0 T
The significance of the upper numerals along the abscissa on Fig. 2 is as follows:
1. Detergent
2. Detergent + Alcalase® 2.0 T (0.05 AU/l)
3. Detergent + Savinase® 4.0 T (0.15 KNPU/l)
4. Detergent + SP 341 (3000 LU/l)
5. Detergent + Granulate I (Alcalase®/SP 341)
6. Detergent + SP 341 + Alcalase® 2.0 T
7. Detergent + Granulate II (Savinase®/SP 341)
8. Detergent + SP 341 + Savinase® 4.0 T
Table 1 shows the total amount of fatty matter left on the swatches after 4 washes.

**Table 1**

| Total residual fatty matter on the swatches after 4 washes. | |
|---|---|
| Trial | Residual fat (% w/w) |
| 1. | 5.31% |
| 2. | 5.67% |
| 3. | 5.03% |
| 4. | 3.69% |
| 5. | 3.77% |
| 6. | 4.55% |
| 7. | 4.71% |
| 8. | 5.15% |

### Comments to Example 1

### Fig. 1

The SP 341 lipase (4) exhibits the major efficacy to remove fats. The combination of Alcalase® encapsulated with a hydrophopbic agent and SP 341 (5) generates nearly the same effect. The combination of conventional Alcalase® and SP 341 (6) only exhibits half of this efficacy. The same trend is seen for the Savinase® case (7-8), but the SP 341 seems to be more sensitive to Savinase® than to Alcalase®.

### Fig. 2

The remission measurements show that the protease performance dominates. In all cases where protease is present the overall performance is close to a remission around 50 units. The SP 341 only shows a marginal effect compared to the detergent alone.

### EXAMPLE 2

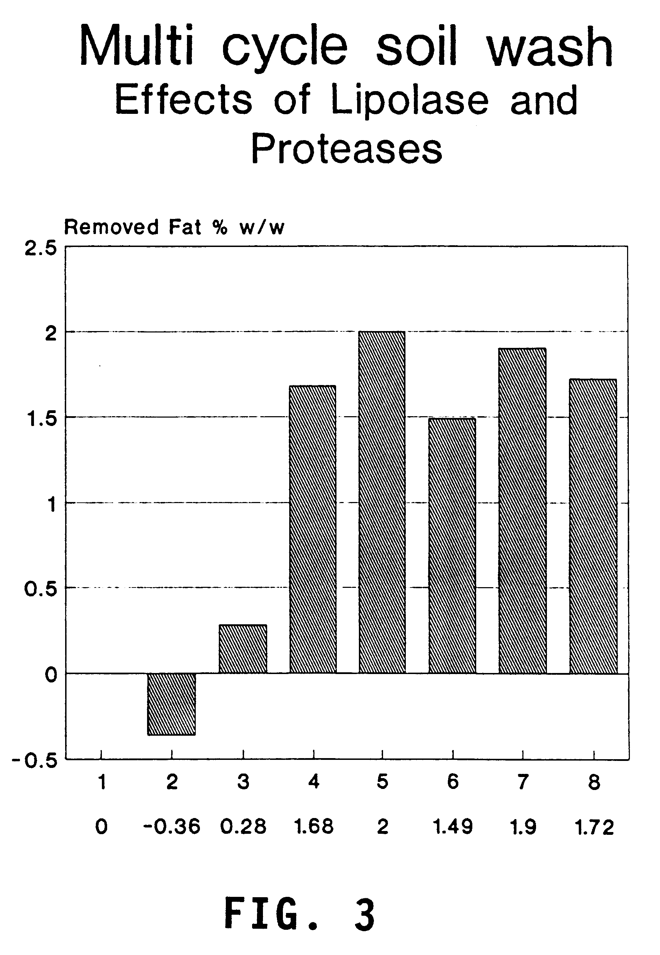

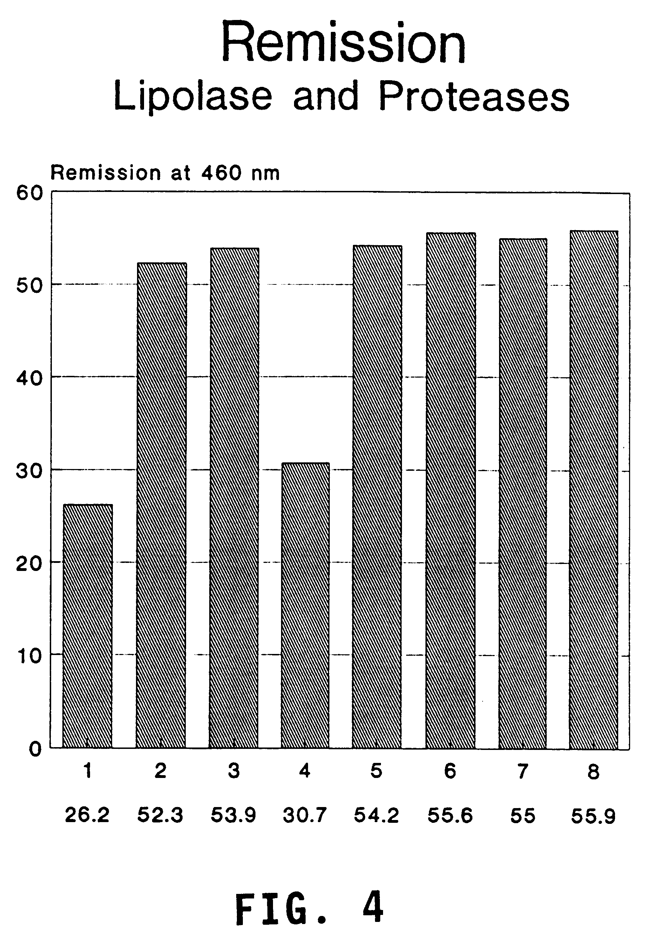

The results from the performance test are listed in Figs. 3 and 4. The efficacy of the lipase is listed in Fig. 3 whereas Fig. 4 shows the overall performance measured as remission of the swatches.

The significance of the upper numerals along the abscissa on Fig. 3 is as follows:
1. Detergent
2. Detergent + Alcalase® 2.0 T (0.05 AU/l)
3. Detergent + Savinase® 4.0 T (0.15 KNPU/l)
4. Detergent + Lipolase® 100 T (3000 LU/l)
5. Detergent + Granulate III (Alcalase®/Lipolase®)
6. Detergent + Lipolase® 100 T + Alcalase® 2.0 T
7. Detergent + Lipolase® 100 T + Savinase® 4.0 T (coated with MSP 90)
8. Detergent + Lipolase® 100 T + Savinase® 4.0 T
The significance of the upper numerals along the abscissa on Fig. 4 is as follows.
1. Detergent
2. Detergent + Alcalase® 2.0 T (0.05 AU/l)
3. Detergent + Savinase® 4.0 T (0.15 KNPU/l)
4. Detergent + Lipolase® 100 T (3000 LU/l)
5. Detergent + Granulate III (Alcalase®/Lipolase®)
6. Detergent + Lipolase® 100 T + Alcalase® 2.0 T
7. Detergent + Lipolase® 100 T + Savinase® 4.0 T (coated with MSP 90)
8. Detergent + Lipolase® 100 T + Savinase® 4.0 T
Table 2 shows the total amount of fatty matter left on the swatches after 4 washes.

**Table 2**

| Total residual fatty matter after 4 washes. | |
|---|---|
| Trial | Residual fat (% w/w) |
| 1. | 5.31% |
| 2. | 5.67% |
| 3. | 5.03% |
| 4. | 3.63% |
| 5. | 3.31% |
| 6. | 3.82% |
| 7. | 3.41% |
| 8. | 3.59% |

### Comments to Example 2

### Fig. 3

Lipolase® is not as sensitive to proteases as SP 341. Still there is observed a small decrease in performance in the trials 6 and 8 where the proteases are released immediately compared to the sustained release coated preparations 5 and 7.

### Fig. 4

Again the overall performance is dominated by the proteases. Lipolase® alone gives a better result than SP 341 in Example 1 but still only marginal efficacy compared to the proteases.

## Claims

1. Enzyme containing granulate comprising a core with a first enzyme and cellulose fibres or artificial fibres in an amount of between 1.5 and 40% by weight, based on the weight of the core, surrounded by a shell comprising a binder, a filler, granulating agents, and a second enzyme, and a sustained release coating between the core and the shell, the second enzyme being sensitive to the first enzyme.

2. Granulate according to Claim 1, wherein the core is of a shape corresponding to a maximum ratio between the largest and the smallest dimension of around 3.

3. Granulate according to Claims 1 - 2, wherein the amount of the core is between 5 and 75% by weight.

4. Granulate according to Claims 1 - 3 wherein the core has a mean particle size between 100 and 1000 »m.

5. Granulate according to Claims 1 - 4, wherein the cellulose fibres or the artificial fibres have an average fibre length of 50-2000 »m and an average fibre width of 5-50 »m.

6. Granulate according to Claims 1 - 5, wherein the shell contains between 1.5 and 40% by weight of cellulose fibres or artificial fibres.

7. Granulate according to Claims 1 - 6, wherein the first enzyme is a protease.

8. Granulate according to Claim 7, wherein the first enzyme is ALCALASE® protease, SAVINASE® protease, and/or ESPERASE® protease and the second enzyme is a lipase.

9. Granulate according to Claim 8, wherein the second enzyme is LIPOLASE® lipase.

10. Granulate according to Claims 1 - 9, wherein the filler consists of or comprises inorganic salts.

11. Granulate according to Claims 1 - 10, wherein the shell is surrounded by a final dust suppressing coating.

12. Granulate according to Claims 1 - 11, wherein the sustained release coating is a material comprising a mono- and/or di- and/or triglyceride of a fatty acid or fatty acids.

13. Method for production of the enzyme containing granulate according to Claims 1-12, which method comprises the introduction of the core into a granulating device, whereafter sequentially the sustained release coating agent, the shell material, and optionally a final dust suppressing coating agent are introduced into the granulating device.

## Patentansprüche

1. Enzymhaltiges Granulat, umfassend einen Kern mit einem ersten Enzym und Cellulosefasern oder künstliche Fasern in einer Menge von 1,5 bis 40 Gew.-%, bezogen auf das Gewicht des Kerns, umgeben von einem Mantel mit einem Gehalt an einem Bindemittel, einem Füllstoff, Granuliermitteln und einem zweiten Enzym, sowie einen Überzug mit Depotwirkung zwischen dem Kern und dem Mantel, wobei das zweite Enzym gegenüber dem ersten Enzym empfindlich ist.

2. Granulat nach Anspruch 1, wobei der Kern eine Gestalt aufweist, die einem maximalen Verhältnis zwischen der größten und der kleinsten Abmessung von etwa 3 entspricht.

3. Granulat nach Anspruch 1-2, wobei der Anteil des Kerns zwischen 5 und 75 Gew.-% liegt.

4. Granulat nach Anspruch 1-3, wobei der Kern eine durchschnittliche Teilchengroße zwischen 100 und 1000 »m aufweist.

5. Granulat nach Anspruch 1-4, wobei die Cellulosefasern oder die künstlichen Fasern eine durchschnittliche Faserlange von 50-2000 »m und eine durchschnittliche Faserbreite von 5-50 »m aufweisen.

6. Granulat nach Anspruch 1-5, wobei der Mantel zwischen 1,5 und 40 Gew.-% Cellulosefasern oder künstliche Fasern enthält.

7. Granulat nach Anspruch 1-6, wobei es sich beim ersten Enzym um eine Protease handelt.

8. Granulat nach Anspruch 7, wobei es sich beim ersten Enzym um ALCALASE^{R}-Protease, SAVINASE^{R}-Protease und/oder ESPERASE^{R}-Protease und beim zweiten Enzym um eine Lipase handelt.

9. Granulat nach Anspruch 8, wobei es sich beim zweiten Enzym um Lipolase^{R}-Lipase handelt.

10. Granulat nach Anspruch 1-9, wobei der Füllstoff aus anorganischen Salzen besteht oder diese umfaßt.

11. Granulat nach Anspruch 1-10, wobei der Mantel von einem staubunterdrückenden Endüberzug umgeben ist.

12. Granulat nach Anspruch 1-11, wobei es sich beim Überzug mit Depotwirkung um ein Material aus Mono- und/oder Di- und/oder Triglycerid einer oder mehrerer Fettsäuren handelt.

13. Verfahren zur Herstellung des enzymhaltigen Granulats nach Anspruch 1-12, wobei das Verfahren die Einführung des Kerns in eine Granuliervorrichtung umfaßt, wonach nacheinander das Mittel für den Überzug mit Depotwirkung, das Mantelmaterial und gegebenenfalls ein Mittel für den staubunterdrückenden Endüberzug in die Granuliervorrichtung eingebracht werden.

## Revendications

1. Granulé contenant un enzyme comportant un coeur ayant un premier enzyme et une quantité de fibres de cellulose ou de fibres artificielles située entre 1,5 et 40% en poids, basée sur le poids du coeur, entouré par une enveloppe comportant un agent de liaison, un agent de remplissage, des agents de granulation, et un second enzyme, et un agent de revêtement à libération prolongée situé entre le coeur et l'enveloppe, le second enzyme étant sensible au premier enzyme.

2. Granulé selon la revendication 1, dans lequel le coeur est d'une configuration correspondant à un rapport maximum entre la plus grande et la plus petite dimension, situé aux alentours de 3.

3. Granulé selon les revendications 1 à 2, dans lequel la quantité de coeur est située entre 5 et 75% en poids.

4. Granulé selon les revendications 1 à 3, dans lequel le coeur a une taille moyenne de particules située entre 100 et 1000 »m.

5. Granulé selon les revendications 1 à 4, dans lequel les fibres de cellulose ou les fibres artificielles ont une longueur moyenne de fibre de 50 à 2000 »m et une largeur moyenne de fibre de 5 à 50 »m.

6. Granulé selon les revendications 1 à 5, dans lequel l'enveloppe contient entre 1,5 et 40% en poids de fibres de cellulose ou de fibres artificielles.

7. Granulé selon les revendications 1 à 6, dans lequel le premier enzyme est une protéase.

8. Granulé selon la revendication 7, dans lequel le premier enzyme est une protéase ALCALASE (marque déposée), une protéase SAVINASE (marque déposée), et/ou une protéase ESPERASE (marque déposée) et le second enzyme est une lipase.

9. Granulé selon la revendication 8, dans lequel le second enzyme est une lipase LIPOLASE (marque déposée).

10. Granulé selon les revendications 1 à 9, dans lequel l'agent de remplissage comporte des sels inorganiques ou est composé de ceux-ci.

11. Granulé selon les revendications 1 à 10, dans lequel l'enveloppe est entourée par un agent de revêtement final supprimant la poussière.

12. Granulé selon les revendications 1 à 11, dans lequel l'agent de revêtement à libération prolongée est un matériel comportant un mono- et/ou un di- et/ou un triglycéride d'acide gras ou d'acides gras.

13. Procédé de production du granulé contenant un enzyme selon les revendications 1 à 12, procédé qui comporte l'introduction du coeur dans un dispositif de granulation, après quoi l'agent de revêtement à libération prolongée, le matériel de l'enveloppe, et de manière optionnelle un agent de revêtement final supprimant la poussière sont introduits de manière séquentielle dans le dispositif de granulation.
